# EUROPEAN PATENT APPLICATION

(11) **EP 4 302 689 A1**
(43) Date of publication of application: **10.01.2024**
(21) Application number: 22397503.8
(22) Date of filing: 04.07.2022
(51) Int. Cl.: A61B 5/097

(54) **SPIROMETRY FILTER AND A SPIROMETRY FILTER-FLOW TRANSDUCER COMBINATION**

(71) Applicant: Medikro Oy, 70800 Kuopio (FI)
(72) Inventor: POHJOLAINEN, Olli-Pekka Ilmari, 70820 Kuopio (FI); LIPPONEN, Mika Petri, 70420 Kuopio (FI); ELORANTA, Tuukka Juhana, 70300 Kuopio (FI)
(74) Representative: Berggren Oy

(57) **Abstract**

The invention relates to a spirometry filter (10), comprising a casing (11) with a wall encapsulating an inlet chamber (12) having inlet opening (13) for receiving the air flow from the patients lungs and an outlet chamber (14) having outlet opening (15) for conducting the air flow to the spirometry flow transducer being connected to the outlet opening (15), and which spirometry filter (10) further comprises filter sheet (16) attached inside the casing (11) between the inlet chamber (12) and outlet chamber (13) air-tightly such that air coming from the input opening flows from the inlet chamber to the outlet chamber through the filter sheet and wherein the filter sheet is a pleated filter sheet (16) having at least one pleat (19). In the spirometry filter according to the present invention the casing (11) comprises an inlet part (17) comprising the inlet chamber (12) and an outlet part (18) comprising the outlet chamber (13), and that the inlet part (17) and outlet part (18) are separate parts of the casing (11) being attached air-tightly to each other by means of a snap fit joint (20). The invention relates also to a spirometry filter-flow transducer combination (30).

## Description

### Technical field

The invention relates to a spirometry filter and a spirometry filter-flow transducer combination.

### Background

A spirometer is a medical instrument being used in determining the movement of air into and out of the patients lungs. Lung diseases such as asthma, bronchitis, and emphysema can be discovered by screenings carried out by spirometer measurements. There are various types of spirometers that use a number of methods for measurement being based on different types of flow transducers. These are, among others pressure transducers, ultrasonic sensors, water gauges etc. Regardless of measurement principle applied in the spirometer, each of them must have a component which receive air flow coming into and out from the patient lungs during the test and which purpose is to provide measurable sample of patient's breathing ability to the spirometer. For instance, for the type spirometer where pressure transducers are used this component is most typically a tube having specific dimensions and shape and that is called a *"flow transducer".*

Both reusable flow transducers and disposable flow transducers exist today and both have their advantages and disadvantages. Disposable flow transducer has been found advantageous since typically the flow transducer itself is simple in construction, and thus its manufacturing costs are low. On the other hand, being reusable the flow transducer need to be carefully cleaned and disinfected after each measurement to prevent contaminations between patients to be examined by using the same reusable flow transducer. Therefore, in many cases, it has been found it more safe and cost-effective to use disposable flow transducers than reusable, especially in case when very simple flow transducers can be used as the case is with the spirometers produced by the applicant.

When reusable flow transducers are used and/or type of measurement where saliva from the patients mouth may not enter in to the flow transducer separate filters (i.e., spirometry filters) must be used. This is the case also today with such other flow transducers in which spirometry filters are not necessary due to the function of the spirometer, among others because of the present pandemic situation (i.e., to not allow bacteria and viruses from the patient to infect health care personnel carrying out the tests in the testing room, and wise versa to infect the patient by bacteria and viruses from the air of the testing room).

The spirometry filters to be appropriate must have ability to filter enough securely all infecting particles (such as bacteria and viruses) from the air coming from the patients lungs and from the air coming into patients lungs from the testing room. On the other hand, such filtration naturally causes resistance to the air flow, and in order to detect the patients ability to blow and inhale air the resistance of flow may not be too high. These basic requirements for the spirometry filters have been set in a spirometer standards EN ISO 26782 and EN ISO 23747 (for Peak Expiratory Flow -measurement).

For the above-mentioned reasons, the presently known filters having appropriate filtration ability and enough low resistance of flow need to be carefully constructed. Thus, especially, when using disposable spirometry filters and/or flow transducers, large number of them is used even within a single day. To keep the make the costs as low as possible, it is utmost importance to have such disposable equipment which price per unit is as low as possible. However, in the presently known spirometry filters there are several parts which need to be connected to each other during their assembly. Especially, the casings and filter sheets need to be heat sealed, glued and/or connected to each other by means of separate attachment elements when the spirometry filters are assembled during their production. Furthermore, the presently known spirometry filters are typically substantially large in size, especially if they are such that they meet the requirements of the spirometry standard in respect of both, the filtration ability and maximum pressure drop. Thus, the presently known spirometry filters and flow transducers as separate components need quite a lot space during their transportation and storage which makes their logistics costs rather high.

### Summary

An aim of the present invention is to achieve novel spirometry filter which has appropriate filtration ability and resistance of flow and which is cheaper and simpler to manufacture than the presently known spirometry filters and which, however, meets the requirements laid down in the above-mentioned spirometry standards. Furthermore, another aim of the present invention is to provide also a spirometry filter-flow transducer combination which is compact in size and cheap to produce and which is suitable for use in a spirometry equipment which is based on measurement of pressure drop that occurs in the flow transducer part of the spirometry filter-flow transducer combination.

The aim of the invention is achieved by the spirometry filter according to the present invention because it has a casing, which comprises two parts, namely an inlet part and an outlet part that are connected to each other by means of snap fit joint so that the filter sheet of the spirometry filter can be attached air-tightly inside the casing by means of that same snap-fit joint. Furthermore, the aim of the invention is achieved by the spirometry filter-flow transducer combination because the spirometry filter-flow transducer combination has flow transducer part that is integral with the filter part and which flow transducer part is based on pressure drop measurement by means of a pneumotach. To put it more precisely, the spirometry filter according to the invention is characterized by what has been presented in the independent claim 1 and spirometry filter-flow transducer combination according to the invention is characterized by what has been presented in the independent claim 11. The dependent claims 2-10 present some advantageous embodiments of the spirometry filter according to the invention. The dependent claims 12-15 present some advantageous embodiments of the spirometry filter-flow transducer combination according to the invention

An advantage of the spirometry filter according to the invention is that regardless of that it meets quality requirements in respect of the filtration ability and resistance of flow (for both inhale breath and exhale blow), set to the spirometry filters in the spirometer standards, the spirometry filter of the present invention is simpler and cheaper to produce because the snap-fit joint between inlet part and outlet part of the casing causes that such additional process phases as gluing or ultrasonic welding is not needed anymore in the assembly of the spirometry filter. Therefore, especially the disposable versions but, of course, also the reusable versions form an economically profitable and competitive alternative compared to the presently known spirometry filters which manufacturing is more complex due to their more complicated assembly methods. Furthermore, because a pleated filter sheet can be used in the spirometry filter according to the invention, it is also easier to make its dimensions smaller. This reduces the logistics and storage costs compared to those known spirometry filters wherein the pleated filter sheets are not used and which are therefore larger in size.

An advantage of the spirometry filter-flow transducer combination according to the present invention is that the space required by a filter-flow transducer combination in both transportation and storage is smaller than that of the separate filters and flow transducers (e.g., because separate filters and flow transducers need to have also individual packages). This makes the filter-flow transducer combinations of the present invention logistically advantageous compared to the presently known separate filters and flow transducers. Also the production costs of the filter-flow transducer combinations are lower because of less separate parts exists which reduces, among others, manufacturing costs in the die-casting since costs in the die-casting highly depends on the amount of separate parts to be die-casted. Furthermore, additional cost savings are reached in the production because number of final product inspections is halved due to a single inspection phase needed for filter-flow transducer combination instead of two separate inspections required for the separate spirometry filters and flow transducers.

### Brief description of the drawings

In the following, the invention is described in more detail with reference to the appended drawings, wherein
- Fig. 1: is a side view of an embodiment of a spirometry filter according to the invention,
- Fig. 2: is the cross-section A-A of the embodiment of the spirometry filter shown in the figure 1,
- Fig. 3: is a top view of the embodiment of the spirometry filter shown in the figures 1 and 2,
- Fig. 4: shows the cross-section B-B of the spirometry filter shown the figures 1 to 3,
- Fig. 5: is a side view of an embodiment of a spirometry filter-flow transducer combination according to the invention,
- Fig. 6: is a top view of the embodiment of the spirometry filter-flow transducer combination show in the figure 5,
- Fig. 7: is a bottom view of the embodiment of the spirometry filter-flow transducer combination show in the figures 5 or 6
- Fig. 8: is a front view of the embodiment of the spirometry filter-flow transducer combination show in the figures 5-7
- Fig. 9: is a rear view of the embodiment of the spirometry filter-flow transducer combination show in the figures 5-8, and
- Fig. 10: is an oblique top view of the embodiment of the spirometry filter-flow transducer combination show in the figures 5-9 when the input part, the filter sheet and the output part have been exploded.

### Detailed description of some advantageous embodiments

The devices described in the present disclosure is intended to be used with spirometry measurement equipment for carrying out pulmonary function tests. The spirometry filter protects the spirometry test operators from viral and bacterial infections when carrying out the tests. The spirometry filter-flow transducer combination forms a single unit which has integral filter part and flow transducer part and which need not to have separate spirometry filter.

Both the spirometry filter and the spirometry filter-sensor combination of the present invention are configured to be compatible at least with a spirometry test equipment wherein the test is based on measurement of pressure drop caused by a pneumotach in a spirometry flow transducer. Thus, the spirometry filter according to the present invention can be detachably attached to any spirometry flow transducer applying the above-described measuring principle. Both the separate spirometry filter and spirometry filter-flow transducer combination according to the present invention can have disposable or reusable versions.

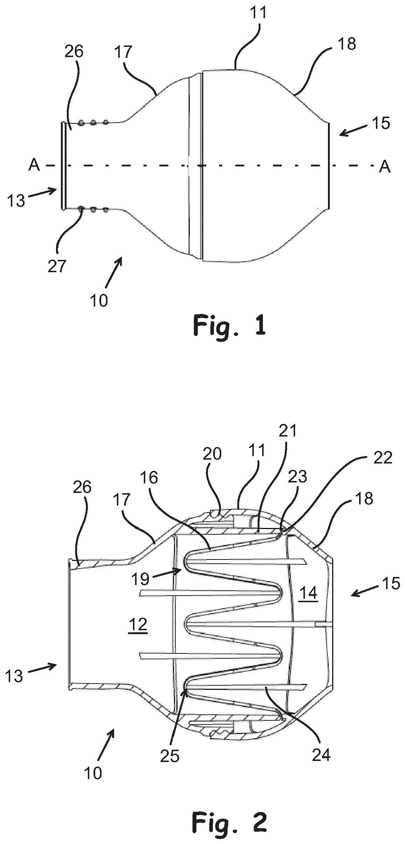

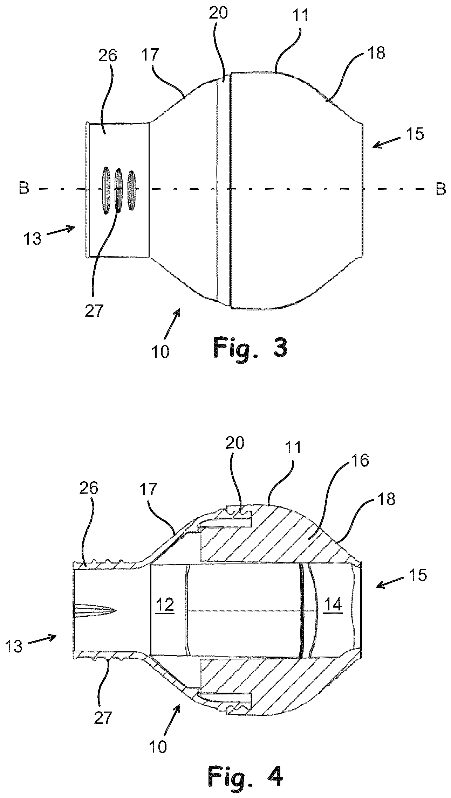

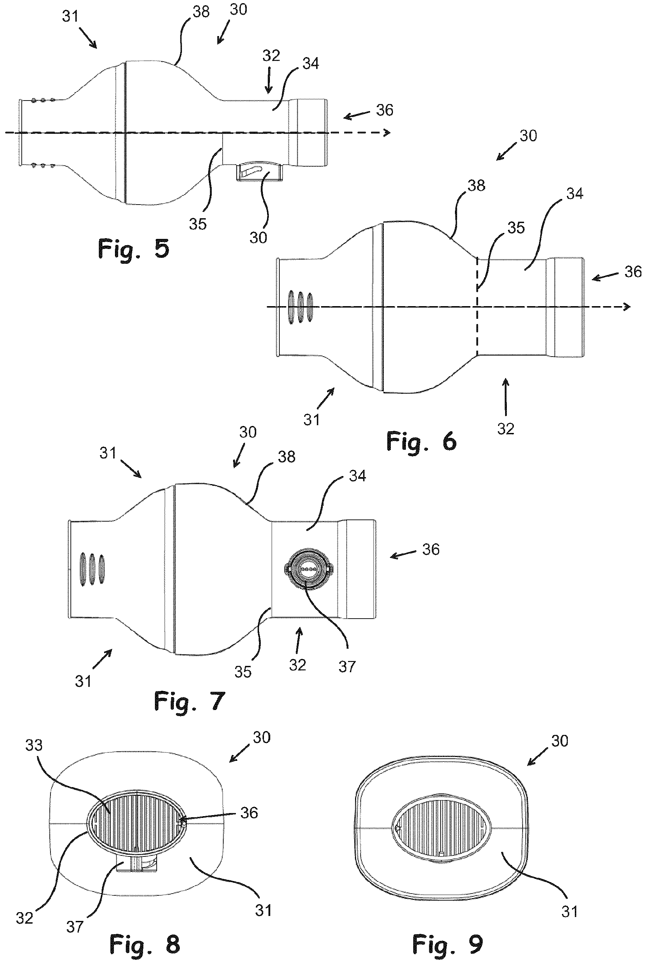

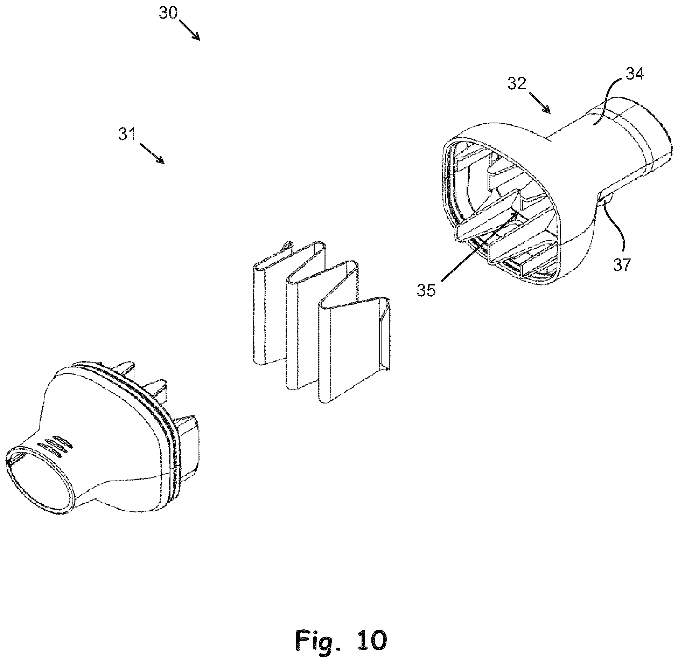

The spirometry filter shown in the figures 1-4 is described hereinbelow as a separate spirometry filter that is detachably attachable to any separate spirometry flow transducer, especially of the type applying the measurement principle described above. Alternatively, it may form the filter part of the filter-flow transducer combination, such as shown in the figures 5-10. In that case it has been constructed within a common body or casing with the flow transducer part (i.e., being integral with the flow transducer part).

The spirometry filter of figures 1-4 comprises a casing 11 with a wall encapsulating an inlet chamber 12 having inlet opening 13 for receiving the air-flow from the patients lungs during the spirometry test and an outlet chamber 14 having outlet opening 15 for conducting the air flow to the spirometry flow transducer being connected to the outlet opening 15. The spirometry filter 10 comprises also filter sheet 16 attached air-tightly inside the casing 11 between the inlet chamber 12 and outlet chamber 13. During the spirometry test the patient blows air in to the spirometry filter 10 through the inlet opening 13 at the mouth piece. Thus, the air entering to the spirometry filter flows from the inlet chamber 12 to the outlet chamber 14 through the filter sheet 16 and therefore the filter sheet 16 remarkably reduces the amount of viruses and bacteria coming from the patients lungs to enter from the inlet chamber 12 to the outlet chamber 13. Since the embodiment of the spirometry filter 10 shown in the figures 1-4 is configured to comply the requirements of the spirometer standard ISO 26782 it has ability to filter enough securely all infecting particles (such as bacteria and viruses) from the air coming from the patients lungs without causing the resistance of flow being not higher than maximum stated in the said spirometry standard. This is achieved by the filter sheet 16 having appropriate filtration properties of the filter sheet material and enough large area of the filter sheet through which the air can flow without causing too high pressure drop. The filter sheet materials can be any fabric materials being used in spirometry filter sheets. Preferably, the material is such that it is as cost-effective as possible but have good enough filtration properties (i.e., such that it appropriately prevents infecting particles to pass through the filter sheet).

Every filter sheet material cause more or less resistance to the air-flow through the spirometry filter. Thus, the area of the filter sheet must be large enough to ensure enough low pressure drop over the filter sheet. To achieve enough large area of the filter sheet without increasing its outer dimensions too much (and thus enlarge the size of the casing 11) the filter sheet 16 is, in the embodiment of figs. 1-4, a pleated filter sheet having several pleats 19. The dimensions of the pleats are preferably such that length to width ratio of the at least one pleat 19 is more than 1. However, in different embodiments of the spirometry filter the number of pleats and their dimensions can vary since the outer dimensions of the spirometry filter can be reduced also by using, e.g., fewer pleats than in the embodiment of figures 1-4. Thus, there may be such embodiments wherein the filter sheet has only one pleat or there may be also such embodiments which has them two or more (like in the filter sheet 16 of the embodiment of figures 1-4). Therefore, in the different embodiments also the shape and size (diameter) of the casing may vary in order to have appropriate space for the filter sheet. Of course, it is advantageous if the casing is as small as possible which makes the spirometry filter less space requiring.

In the embodiment of figs 1-4, the casing 11 is formed of two separate parts; namely an inlet part 17 and outlet part 18. The inlet part 17 comprises the inlet chamber 12 and the outlet part 18 comprises the outlet chamber 13. The inlet part 17 and outlet part 18 are attached air-tightly to each other by means of a snap fit joint 20. By the inlet chamber 12, it is meant an inner space being limited by the casing wall of the inlet part 17 and the filter sheet 16. The inlet part 17 comprises also mouth piece 26 which is integral with the inlet part 17. The mouth piece 26 has teeth protrusions 27 by means of which the patient can hold the mouth piece in her/his mouth when blowing air in to the filter during the test. Thus, the inlet chamber 12 is a part of the casing 11 in to which the air enters through the inlet opening 13 from the patients mouth when the spirometer filter 10 is used. By the outlet chamber 14, it is meant an inner space limited by the filter sheet 16 and the casing wall of the outlet part 18. Thus, the outlet chamber 14 is a part of the casing 11 from which the air exits through the outlet opening 15 when the spirometer filter 10 is used. The material of the casing 11 is preferably some biocompatible plastic, such as PEHD plastic.

In the embodiment of the spirometry filter according to figures 1-4 the filter sheet 16 is secured air-tightly to the wall of the casing 11 by means of the snap fit joint 20. In such attachment, an outer edge 23 of the filter sheet 16 may be compressed between abutting edges of the inlet part 17 and the outlet part 18. This can be accomplished e.g., by such a way that the inlet part 17 and the outlet part 18 comprise sealing protrusions 21 and 22 arranged to abut against each other as shown in the figure 2 when the inlet part 17 and the outlet part 18 have been connected to each other. However, other arrangements may be also possible. Thus, the outer edge may be attached e.g., between some specific portions of the snap fit joint 20. An advantage of this arrangement is that filter sheet 16 can be attached in to the casing 11 tightly and firmly without any separate attachment methods such as ultrasonic welding or gluing. Thus, such arrangement makes the manufacturing process of spirometry filter 10 simpler and cheaper. In addition that application of above-mentioned separate attachment methods are avoided this is also because the attachment of the filter sheet 16 can be carried out simultaneously with the assembly of the casing 11 i.e., without any separate process phase when the inlet part 17 and outlet part 18 are connected to each other by the snap fit joint 20.

In an embodiment of the spirometry filter shown in the figures 1-4 the spirometry filter 10 includes pleat supports 24. The aim of the pleat supports 24 is to ensure that the filter sheet 16 remains its original position and is not disturbed when the air blown by a patient flows through the spirometry filter during the spirometry test. The pleat supports 24 are, in this embodiment, plate-like elements which have been arranged to support the filter sheet 16 at the support positions 25 which preferably located at the positions where the filter sheet 16 has been folded to form the pleats 19. As shown in the figure 2 the pleat supports 24 have been directed parallel to the direction of the air-flow. Thus, as a thin plate-like elements, they have minimal effect on the air-flow but are very rigid in the direction of the load caused by the filter sheet 16 during the time when the spirometry test is carried out.

The number of pleat supports 25 preferably depends on the number of the pleats 19. In the embodiment of figures 1-4 the number of pleat supports 25 is equal to number of pleats 19. However, there may be also such embodiments wherein each pleat does not have their own a pleat support. Thus, there may be, e.g., such embodiments which have one pleat for each two pleats, or even such embodiments which has even only one pleat support for the whole filter sheet. Of course, in the last mentioned case, the pleat support is preferably placed at the position of a pleat that is in the middle of the inner of the casing.

The figures 5-10 shows an embodiment of a filter-flow transducer combination according to the invention. The filter-flow transducer combination 30 shown in the figures 5-10 comprises filter part 31 and flow transducer part 32 that have been integrated together so that they form a single unit. In the filter-flow transducer combination 30 the flow transducer part 32 comprises a pneumotach 33 for causing measurable pressure drop within the flow transducer part 32 when air is blown through the spirometry filter-flow transducer combination 30.

The flow transducer part 32 comprises a tube 34 having inlet opening 35 for receiving air flow from the filter part 31 and an outlet opening 36 through which the air flow exits from inside the spirometry filter-flow transducer combination 30. The flow transducer part 32 comprises also a connection port 37 between the inlet opening 35 and the outlet opening 36 for connecting a pressure drop gauge to the flow transducer part 32. The inlet opening 35 is inside the common casing 38 for the both filter part 31 and the flow transducer part 32. However, in the embodiment shown in the figures 5-10 the filter part 31 has separate inlet part that is connected to the rest of the casing by a snap fit joint such as described above in connection with the spirometry filter shown in the figures 1-4.

The pneumotach 33 is an element in the flow transducer part 32 that causes a measurable pressure drop to the air-flow flowing through the tube 34 of the flow transducer part 32. The pneumotach 33 locates in the tube 34 after the connection port 37. Thus the pressure drop caused by the pneumotach 33 can be measured by measuring the air pressure in the connection port 37 that is located before the pneumotach 33. In the embodiment shown in the figures 5-10 the pneumotach 33 is a grating that resist the air flow flowing through the tube 34 in a known manner. Thus, it causes that air-pressure during the time when the patient blows in the portion of the flow transducer part being before the pneumotach 33 is higher than in portion after the pneumotach 33.

The filter part 31 comprises a spirometry filter which prevents possible viruses and bacteria from entering in to the flow transducer part 32 and through there to the surrounding environment. Thus, the aim of the filter portion 31 is to protect the operator carrying out the measurements from being infected by the viruses and/or bacteria coming from the lungs of the patient during the test. The filter part 31 may be formed of e.g., a spirometry filter that corresponds structurally and functionally the spirometry filter 10 of the figures 1-4 described above. In such case the outlet opening 15 of the spirometry filter 10 (i.e., the filter part 31) coincide with the inlet opening 35 of the flow transducer part 32.

Alternatively the filter part may be formed of any other kind of spirometry filter construction being suitable for filtering viruses and/or bacteria according to above-mentioned spirometry standards and which can be used at least with the flow transducer part of the type described above. Thus, the spirometry filter and spirometry filter-flow transducer combination are not limited to the embodiments described above but can vary within the scope of the appended claims.

## Claims

1. A spirometry filter (10), comprising a casing (11) with a wall encapsulating an inlet chamber (12) having inlet opening (13) for receiving the air flow from the patients lungs and an outlet chamber (14) having outlet opening (15) for conducting the air flow to the spirometry flow transducer being connected to the outlet opening (15), and which spirometry filter (10) further comprises filter sheet (16) attached inside the casing (11) between the inlet chamber (12) and outlet chamber (14) air-tightly such that air coming from the input opening flows from the inlet chamber to the outlet chamber through the filter sheet and wherein the filter sheet is a pleated filter sheet (16) having at least one pleat (19), **characterized in that**, the casing (11) comprises an inlet part (17) comprising the inlet chamber (12) and an outlet part (18) comprising the outlet chamber (13), and that the inlet part (17) and outlet part (18) are separate parts of the casing (11) being attached air-tightly to each other by means of a snap fit joint (20).

2. The spirometry filter (10) according to claim 1 wherein the filter sheet (16) is secured air-tightly to the wall of the casing (11) by means of the snap fit joint (20).

3. The spirometry filter (10) according to claim 2 wherein an outer edge of the filter sheet (16) has been compressed between abutting edges of the inlet part (17) and the outlet part (18).

4. The spirometry filter (10) according to any of preceding claims wherein the inlet part (17) and the outlet part (18) comprise sealing protrusions (21, 22) arranged to abut against each other when the inlet part (17) and the outlet part (18) have been connected to each other.

5. The spirometry filter (10) according to claim 4 wherein the dimensions of the snap fit joint (20) of the inlet part (17) and the outlet part (18) has been designed such that an outer edge (23) of the filter sheet (16) being fitted between the sealing protrusions (21, 22) is compressed by the sealing protrusions (21, 22).

6. The spirometry filter (10) according to any of preceding claims wherein the spirometry filter (10) includes at least one pleat support (24) being arranged to support the filter sheet (16) at the support position (25) wherein the filter sheet (16) has been folded to form the at least one pleat (19) to the filter sheet (16).

7. The spirometry filter (10) according to any of preceding claims wherein length to width ratio of the at least one pleat (19) is more than 1.

8. The spirometry filter (10) according to any of preceding claims wherein the filter sheet (16) includes two or more pleats (19).

9. The spirometry filter (10) according to any of preceding claims wherein the spirometry filter (10) includes a mouth piece (26) being integral with the spirometry filter (10).

10. The spirometry filter according to claim 9 wherein the mouthpiece (26) includes at least one teeth protrusion (27).

11. A spirometry filter-flow transducer combination (30), comprising filter part (31) and flow transducer part (32), **characterized in that**, the filter part (31) and the flow transducer part (32) are integrated together, and that the flow transducer part (32) comprises a pneumotach (33) for causing measurable pressure drop within the flow transducer part (32) when air is blown through the spirometry filter-flow transducer combination (30).

12. The spirometry filter-flow transducer combination (30) according to claim 11 wherein the flow transducer part (32) comprises a tube (34) having inlet opening (35) for receiving air flow from the filter part (31) and an outlet opening (36) for exiting the air flow from the spirometry filter-flow transducer combination (30).

13. The spirometry filter-flow transducer combination (30) according to claim 12 wherein the flow transducer part (32) comprises a connection port (37) between the inlet opening (35) and the outlet opening (36) for connecting a pressure drop gauge to the flow transducer part (32).

14. The spirometry filter-flow transducer combination (30) according to claim 13 wherein the pneumotach (33) of the flow transducer part (32) is a grating being placed downstream to the connection point (37).

15. The spirometry filter-flow transducer combination (30) according to any of claims 11 to 14 wherein the filter part (31) comprises a spirometry filter (10) according to any of claims 1 to 10.
